(19) Europäisches Patentamt
European Patent Office
Office européen des brevets

(11) Veröffentlichungsnummer : **0 168 563**
**B1**

(12) # EUROPÄISCHE PATENTSCHRIFT

(45) Veröffentlichungstag der Patentschrift : **08.07.87**

(21) Anmeldenummer : **85104952.8**

(22) Anmeldetag : **24.04.85**

(51) Int. Cl.[4] : **C 07 C121/413**, C 07 C 69/593, C 07 C 69/618, C 07 B 41/12, C 07 B 43/08, C 07 C120/00, C 07 C 67/343

(54) **Verfahren zur Herstellung von Alkyliden- und Arylidenverbindungen.**

(30) Priorität : **23.06.84 DE 3423249**

(43) Veröffentlichungstag der Anmeldung : **22.01.86 Patentblatt 86/04**

(45) Bekanntmachung des Hinweises auf die Patenterteilung : **08.07.87 Patentblatt 87/28**

(84) Benannte Vertragsstaaten : **CH DE FR GB LI**

(56) Entgegenhaltungen :
**DE-A- 2 042 610**
**DE-B- 1 468 194**
**GB-A- 1 048 906**
**CHEMICAL ABSTRACTS, Band 100, Nr. 23, 4. Juni 1984, Seite 547, Nr. 191500d, Columbus, Ohio, US; ATTANASI, ORAZIO et al.: "Effect of metal ions in organic synthesis. Part XVI. Knoevenagel condensations of aldehydes and tosylhydrazones with 2,4-pentanedione by copper(II) chloride-catalyzed reaction"**
**TETRAHEDRON LETTERS, Band 23, Nr. 47, 1982, Seiten 4927-4928 Pergamon Press Ltd., GB; F. TEXIER-BOULLET et al.: "Knoevenagel condensation catalysed by aluminium oxide"**
**ORGANIC REACTIONS, Band 15, 1973, Seiten 204-597, John Wiley & Sons, Inc.; G. JONES: "The Knoevenagel condensation"**

(73) Patentinhaber : **HÜLS AKTIENGESELLSCHAFT**
**- RSP Patente / PB 15 - Postfach 13 20**
**D-4370 Marl 1 (DE)**

(72) Erfinder : **Kleine Homann, Walter, Dr.**
**Buchenallee 14**
**D-4408 Dülmen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 168 563 B1**

Jouve, 18, rue St-Denis, 75001 Paris, France

**Beschreibung**

Alkyliden- und Arylidenverbindungen erhält man nach der Knoevenagel-Reaktion durch Kondensation einer Carbonylkomponente mit CH-aciden Methylenverbindungen, z. B. Acetaldehyd mit Malonsäurediethylester. Als Katalysator fungieren Amine wie beispielsweise Piperidin oder Alanin in Gegenwart von Eisessig.

$$CH_3CHO + CH_2(COOR)_2 \xrightarrow{Kat.} CH_3{-}CH{=}C(COOR)_2$$

(Organic Reactions Vol. 15, 204-597, John Wiley & Sons Inc. 1967).

Als weitere Verbindung entsteht häufig das decarboxylierte Produkt. (Henecka in Houben Weyl, Bd. 8, 1952, S. 365-502).

Zur Verschiebung des Gleichgewichtes wird empfohlen, das Reaktionswasser durch Schleppmittel wie Benzol, Toluol oder Chloroform auszukreisen. Stärker basische Katalysatoren wie Natrium- oder Kaliumacetat führen durch Telomerisation zu überproportionaler Nebenproduktbildung (J. Org. Chem. 38, 1512, 1973).

Es besteht daher ein Interesse an einem Verfahren, bei dem einerseits der Einsatz besonderer Extraktionsmittel nicht erforderlich ist, andererseits eine überproportionale Nebenproduktbildung vermieden wird.

Die sich hieraus ergebende Aufgabe wird erfindungsgemäß entsprechend den Angaben der Patentansprüche gelöst.

Überraschenderweise erhält man Alkyliden- und Arylidenverbindungen in guter Ausbeute durch Umsetzung der entsprechenden CH-aciden Verbindungen mit gesättigten aliphatischen oder araliphatischen Carbonylverbindungen mit 1 oder mehr C-Atomen, wenn man die Reaktion in Gegenwart einer Metallverbindung eines Metalls der II., III. oder IV. Hauptgruppe oder der I., II., VI., VII. oder VIII. Nebengruppe sowie deren Mischungen und in Gegenwart von 1 bis 40 % Wasser, bezogen auf die Metallverbindung durchführt. Die in Gegenwart von Natrium- oder Kaliumacetat erfolgende überproportionale Nebenproduktbildung tritt bei den erfindungsgemäßen Metallverbindungen nicht ein.

Bezogen auf die CH-acide Verbindung wird eine Katalysatorkonzentration von 0,001 bis 2 Mol-%, vorzugsweise von 0,01 bis 0,7 Mol-%, angewendet. Geringere Katalysatorkonzentrationen führen zu ungünstigeren Raum-Zeit-Ausbeuten, höhere Konzentrationen bei der Aufarbeitung zu Problemen.

In Gegenwart von 1 bis 40 % Wasser, vorzugsweise 10 bis 30 %, bezogen auf die Metallverbindung, wird das Reaktionsverhalten entgegen den Erwartungen positiv beeinflußt. Die Reaktionsgeschwindigkeit wird erhöht.

Die Umsetzung erfolgt bereits bei niedrigen Temperaturen, beispielsweise bei 10 bis 150 °C. Bevorzugt arbeitet man bei Temperaturen von 40 bis 100 °C. Die Reaktionsführung ist auch bei höheren oder tieferen Temperaturen möglich. Bei tieferen Temperaturen verläuft die Reaktion jedoch langsamer, bei höheren Temperaturen tritt eine merkliche Steigerung der Nebenproduktbildung auf.

Im allgemeinen arbeitet man bei Normaldruck, man kann aber auch bei Unter- oder Überdruck arbeiten. Zur Reaktion wird allgemein die Carbonylkomponente mit dem Katalysator vorgelegt und die CH-acide Verbindung in das temperierte Medium dosiert. Werden alle Reaktionskomponenten gemeinsam zum Temperieren vorgelegt, so ist mit negativen Einflüssen bezüglich der Selektivität des Reaktionsverlaufes zu rechnen.

Geeignete CH-acide Verbindungen sind beispielsweise Malonsäure, Malonsäureester, Malonsäuredinitril, Cyanessigester und aliphatische Nitroverbindungen wie Nitromethan.

Nach beendeter Reaktion kann das Rohprodukt durch einfache Vakuumfraktionierung isoliert werden. Abgeschiedenes Reaktionswasser kann gegebenenfalls inklusive der darin enthaltenen Katalysatorreste vorher abgetrennt werden. Ist eine Isolierung des Produktes nicht erforderlich, so kann die Konzentration an Metallverbindungen erforderlichenfalls durch übliche Verfahren wie Wäschen oder Einsatz von Ionenaustauschern auf den gewünschten Restgehalt reduziert werden.

Als Carbonylverbindungen können Aldehyde oder Ketone eingesetzt werden wie beispielsweise Acetaldehyd, Propionaldehyd, Butyraldehyd, Valeraldehyd, 2-Methylvaleraldehyd, Capronaldehyd, Laurylaldehyd, 2-Phenylpropanal, 3-Phenylpropanal oder Aceton, Methylethylketon, Methylbutylketon, Cyclohexanon und 4-tert.-Butylcyclohexanon.

Als Katalysatoren sind Metallverbindungen eines Metalls der II., III. oder IV. Hauptgruppe oder der I., II., VI., VII. oder VIII. Nebengruppe wie beispielsweise die Acylate wie Acetat oder Propionat des Calciums, Aluminiums, Bleis, Kupfers, Zinks, Cadmiums, Chroms, Mangans, Eisens, Cobalts oder Nickels und Chloride des Zinks oder Eisens und Oxide des Zinks oder Mangans wie auch deren Mischungen geeignet. Bevorzugt setzt man Zinverbindungen, wie Zinkacylate, insbesondere Zinkacetat und/oder Zinkpropionat sowie Cobalt- und/oder Cadmium-Verbindungen, bevorzugt die Acylate, ein. Mit diesen Katalysatoren erhält man hohe Umsätze bei guten Ausbeuten.

Ein wiederholter Einsatz des Katalysatorsystems läßt keine signifikante Desaktivierung erkennen.

Vorteilhaft für die Reaktionsgeschwindigkeit ist es, der Reaktionslösung 10 bis 30 % Wasser, bezogen auf die eingesetzte Katalysatormenge zuzugeben. Dieses kann sowohl über freies Wasser als

auch über Kristallwasser erfolgen. Auch die Verwendung größerer Wasserdosierungen ist möglich. Sie führt jedoch zu keiner weiteren signifikant positiven Beeinflussung.

## Beispiele

In einem Dreihalskolben mit aufgesetztem Kühler werden 1 Mol der Carbonylkomponente und 0,02 Mol des Katalysators auf Reaktionstemperatur erwärmt und innerhalb von 8 h mit einem Mol der CH-aciden Verbindung versetzt. Nach beendeter Reaktion wird das Reaktionswasser abgetrennt und die Reaktionslösung destillativ im Vakuum aufgearbeitet.

In der folgenden Tabelle beziehen sich Umsatz und Ausbeute auf die CH-acide Komponente.

(Siehe Tabelle Seite 4 f.)

| | Carbonylverbindung | CH-acide Komp. | Reaktionsprodukt | Katalysator | Reakt. Temp. °C | Um-satz % | Aus-beute % |
|---|---|---|---|---|---|---|---|
| 1 | Acetaldehyd | Cyanessigester | Ethylidencyanessigester | Calciumacetat | | 90 | 68 |
| 2 | Propionaldehyd | Malonsäuredinitril | Propylidenmalonsäuredinitril | Zinkpropionat | 60 | 96 | 92 |
| 3 | Butyraldehyd | Nitromethan | Nitropenten | Zinkoxid | 80 | 76 | 22 |
| 4 | Valeraldehyd | Malonsäureester | Pentylidenmalonsäureester | Zinkacetat | 60 | 89 | 91 |
| 5 | Capronaldehyd | Malonsäure | Hexylidenmalonsäure | Zinkchlorid | 80 | 88 | 41 |
| 6 | Laurylaldehyd | Malonsäureester | Dodecylidenmalonsäureester | Eisenhydroxiacetat | 80 | 45 | 28 |
| 7 | 2-Phenylpropanal | Malonsäureester | 2-Phenylpropylidenmalonsäureester | Nickelacetat | 100 | 83 | 94 |
| 8 | 3-Phenylpropanal | Malonsäureester | 3-Phenylpropylidenmalonsäureester | Manganacetat | 100 | 79 | 89 |
| 9 | Aceton | Malonsäuredinitril | Isopropylidenmalonsäuredinitril | Cadmiumacetat | 40 | 100 | 68 |
| 10 | Methylethylketon | Cyanessigester | 2-Methylpropylidencyanessigester | Zinkacetat | 60 | 92 | 78 |
| 11 | Methylbutylketon | Malonsäuredinitril | 2-Methylbutylidenmalonsäuredinitril | Bleiacetat | 80 | 78 | 64 |
| 12 | Cyclohexanon | Cyanessigester | Cyclohexylidencyanessigester | Cobaltacetat | 100 | 75 | 94 |
| 13 | 4-tert.-Butyl-cyclohexanon | Cyanessigester | 4-tert.-Butylcyclohexylidencyan essigester | Zinkacetat | 100 | 72 | 93 |
| 14 | Butyraldehyd | Malonsäureester | Butylidenmalonsäureester | Kupferacetat | 70 | 46 | 85 |
| 15 | Butyraldehyd | Malonsäureester | Butylidenmalonsäureester | Chromacetat | 70 | 38 | 52 |
| 16 | Butyraldehyd | Malonsäureester | Butylidenmalonsäureester | Aluminiumacetat | 70 | 26 | 54 |

Entsprechend der vorherigen Versuchsbeschreibung wird Butyraldehyd unter dem katalytischen Einfluß von Zinkacetat mit Malonsäureester zur Reaktion gebracht. Der Einfluß verschiedener Wasserkonzentrationen (bezogen auf den Katalysator) auf die Reaktionsgeschwindigkeit ist aus der folgenden Tabelle zu ersehen. Bezugspunkt ist ein Produktgehalt im Reaktionsgemisch von gleicher Konzentration.

| | Wassergeh. (%) | Reaktions-Temp. (°C) | Reaktionszeit (h) |
|---|---|---|---|
| 17 | < 1 | 60 | 24 (Vergleichsversuch) |
| 18 | 18 | 60 | 7 |
| 19 | 30 | 60 | 9 |
| 20 | 70 | 60 | 19 |

**Patentansprüche**

1. Verfahren zur Herstellung von Alkyliden- und Aryliden-Verbindungen durch Umsetzung der entsprechenden CH-aciden Verbindungen mit gesättigten aliphatischen oder araliphatischen Carbonylverbindungen mit 2 oder mehr C-Atomen, dadurch gekennzeichnet, daß man die Umsetzung in Gegenwart einer Metallverbindung eines Metalls der II., III. oder IV. Hauptgruppe oder der I., II., VI., VII. oder VIII. Nebengruppe sowie deren Gemische und in Gegenwart von 1 bis 40 % Wasser, bezogen auf die Metallverbindung durchführt.

2. Verfahren nach Anspruch 1, dadurch gekennzeichnet, daß man die Umsetzung in Gegenwart von 10 bis 30 % Wasser, bezogen auf die Metallverbindung, durchführt.

3. Verfahren nach Anspruch 1 und 2, dadurch gekennzeichnet, daß man als Metallverbindung Zink-, Cobalt- und/oder Cadmiumverbindungen, bevorzugt die Acylate, einsetzt.

4. Verfahren nach Anspruch 3, dadurch gekennzeichnet, daß man als Zinkacylate Zinkacetat und/oder Zinkpropionat einsetzt.

5. Verfahren nach den Ansprüchen 1 bis 4, dadurch gekennzeichnet, daß man Zinkacylate, vorzugsweise Zinkacetat und/oder Zinkpropionat mit einem Wassergehalt von 10 bis 20 % einsetzt.

**Claims**

1. A process for the production of an alkylidene or arylidene compound by reaction of the corresponding CH-acid compound with a saturated aliphatic or araliphatic carbonyl compound of 2 or more carbon atoms, characterised in that the reaction is carried out in the presence of a metal compound of a metal of main group II, III or IV or a transition metal of sub-group I, II, VI, VII or VIII or a mixture of two or more thereof and in the presence of 1 to 40 % of water, based on the metal compound.

2. A process according to claim 1, characterised in that the reaction is carried out in the presence of 10 to 30 % water, based on the metal compound.

3. A process according to claim 1 or 2, characterised in that a zinc, cobalt and/or cadmium compound, preferably an acylate, is introduced as the metal compound.

4. A process according to claim 3, characterised in that zinc acetate and/or zinc propionate is introduced as the zinc acylate.

5. A process according to any of claims 1 to 4, characterised in that a zinc acylate, preferably zinc acetate and/or zinc propionate, having a water content of 10 to 20 % is introduced.

**Revendications**

1. Procédé de préparation de composés alkylidéniques et arylidéniques par réaction des composés CH-acides correspondants sur des composés carbonylés saturés, aliphatiques ou araliphatiques, comportant 2 atomes de carbone ou davantage, caractérisé par le fait que l'on effectue la réaction en présence d'un composé métallique d'un métal du groupe principal II., III. ou IV. ou du sous-groupe I., II., VI., VII. ou VIII., ainsi que leurs mélanges, et en présence de 1 à 40 % d'eau, relativement au composé métallique.

2. Procédé selon la revendication 1, caractérisé par le fait que l'on effectue la réaction en présence de 10 à 30 % d'eau, relativement au composé métallique.

3. Procédé selon les revendications 1 et 2, caractérisé par le fait que l'on utilise, comme composé métallique, des composés de zinc, de cobalt et/ou de cadmium, de préférence les acylates.

4. Procédé selon la revendication 3, caractérisé par le fait qu'on utilise, comme acylates de zinc, l'acétate de zinc et/ou le propionate de zinc.

5. Procédé selon les revendications 1 à 4, caractérisé par le fait qu'on utilise des acylates de zinc, de préférence l'acétate de zinc et/ou le propionate de zinc, d'une teneur en eau de 10 à 20 %.